# FASCICULE DE BREVET EUROPEEN

(11) **EP 2 819 655 B1**
(45) Date de publication et mention de la délivrance du brevet: **27.07.2016**
(21) Numéro de dépôt: 13715263.3
(22) Date de dépôt: 25.02.2013
(51) Int. Cl.: A61K 9/16, A61K 9/20, A61K 9/48, A61K 31/165, A61P 25/36

(54) **MODAFINIL POUR SON UTILISATION DANS LE TRAITEMENT DES COCAÏNOMANES**
MODAFINIL ZUR ANWENDUNG IN DER BEHANDLUNG VON KOKAINSUCHT
MODAFINIL FOR USE IN THE TREATMENT OF COCAINE ADDICTS

(30) Priorité: 28.02.2012 FR 1200581
(43) Date de publication de la demande: 07.01.2015
(73) Titulaire: Debregeas Et Associes Pharma, 75008 Paris (FR)
(72) Inventeur: SUPLIE, Pascal, F-27400 Montaure (FR); VIVET, Philippe, F-75012 Paris (FR)
(74) Mandataire: Gallochat, Alain
(86) Numéro de dépôt international: PCT/FR2013/000052
(87) Numéro de publication internationale: WO 2013/128088

(56) Documents cités:
- WO-A1-02/30414
- WO-A2-2007/013962
- US-A- 5 618 845
- US-A1- 2006 024 370
- US-A1- 2007 275 057
- HERMAN B H ET AL: "Medications for the treatment of cocaine addiction: Emerging candidates", DRUG DISCOVERY TODAY: THERAPEUTIC STRATEGIES, ELSEVIER, AMSTERDAM, NL, vol. 2, no. 1, 1 avril 2005 (2005-04-01), pages 87-92, XP004991438, ISSN: 1740-6773, DOI: 10.1016/J.DDSTR.2005.05.014
- WONG Y N ET AL: "A double-blind, placebo-controlled, ascending-dose evaluation of the pharmacokinetics and tolerability of modafinil tablets in healthy male volunteers", JOURNAL OF CLINICAL PHARMACOLOGY, LIPPINCOTT CO, HAGERSTOWN, MD, US, vol. 39, no. 1, 1 janvier 1999 (1999-01-01), pages 30-40, XP009022920, ISSN: 0091-2700, DOI: 10.1177/00912709922007534
- DONOVAN J L ET AL: "CHIRAL ANALYSIS OF D- AND L-MODAFINIL IN HUMAN SERUM: APPLICATION TO HUMAN PHARMACOKINETIC STUDIES", THERAPEUTIC DRUG MONITORING, LIPPINCOTT WILLIAMS AND WILKINS, NEW YORK, NY, US, vol. 25, no. 2, 1 janvier 2003 (2003-01-01), pages 197-202, XP008055828, ISSN: 0163-4356, DOI: 10.1097/00007691-200304000-00009

## Description

Le modafinil est le 2-[(diphénylméthyl)sulfinyl)]acétamide, dont la formule moléculaire est C15H15NO2S et la formule développée :

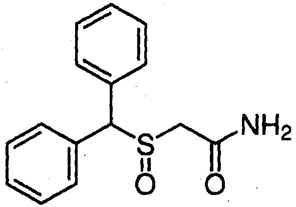

Actuellement, l'utilisation de cocaïne est cause de nombreux troubles médicaux et complications psychiatriques telles que la dépendance. Ainsi la dépendance à la cocaïne est un grave problème de santé publique qui ne fait que croître. Il est décrit aux Etats-Unis que plus de 200 000 personnes en 2002 ont dû subir des soins d'urgence suite à des problèmes médicaux ou psychiatriques consécutifs à l'usage de cocaïne. Aujourd'hui, de nombreuses molécules sont susceptibles de pouvoir être utilisées dans le traitement de l'addiction à la cocaïne. En effet, le mode d'action de la cocaïne au niveau cérébral fait intervenir au moins trois neuromédiateurs : GABA (Gamma aminobutyric acid), glutamate, et DA (dopamine). Ainsi, différentes molécules actives sur ces médiateurs ont fait l'objet d'étude d'efficacité chez l'homme. Ces molécules sont le baclofène, le topiramate, le modafinil, le disulfiram [BH Herman, A. Elkashef, F. Vocci -Medications for the treatment of cocaine addiction : Emerging candidates. Drug Discovery Today : Therapeutic Stratégies, Vol.2, N°1, 2005], [P. Tahsili-Fahadan, GV Carr, GC Harris, and G. Aston-Jones - Modafinil bloks reinstatement of extinguished opiate-seeking in rats: mediation by a glutamate mechanism, Neuropsychopharmacology, 25, 2010, 2203-2210], [J.M. Martinez-Raga; C. Knecht and S. Cepeda - Modafinil: a useful médication for cocaine addiction? Review of the evidence from neuropharmacological, experimental and clinical studies - Current Drug Abuse Reviews, 2008, 1, 213-221].

Le modafinil est un médicament éveillant utilisé en Europe depuis les années 90: il augmente les niveaux d'éveil et de vigilance diurne et est prescrit dans le traitement de la narcolepsie.

Le mode d'action n'est pas totalement connu mais il intervient sur les transmissions adrénergiques et glutamatergiques. Il se lie aux transporteurs de la dopamine et en diminue la recapture.

Il provoque également une inhibition de la recapture de la noradrénaline au niveau du noyau ventrolatéral optique responsable de l'induction du sommeil.

Le modafinil est commercialisé sous les noms de Modiodal, Provigil et Alertec. La dose administrée varie d'une prise de 100 mg à deux prises de 200 mg par jour ; la demi-vie d'élimination est d'environ 14 heures chez l'homme.

Le modafinil est commercialisé sous sa forme racémique qui présente un centre chiral qui est en fait un atome de soufre. Il existe cependant deux isomères optiquement actifs : l'énantiomère dextrogyre (S) et la forme lévogyre (R), ces deux formes étant a priori présentes en quantité égale dans le racémique.

Les deux énantiomères ont la même activité pharmacologique chez l'animal ; cependant, chez l'homme, l'énantiomère R présente une demi-vie de 10 à 14 heures. L'énantiomère S présente, quant à lui, une demi-vie de 3 à 4 heures [Réf. biblio : Wong et al., J.Clin. pharmacol.,39 :30-40(1999) ; Wong et al., J.Clin. pharmacol.,39 :281-288(1999) ; Robertson et al., Clin Pharmacokinet.,42 :123-137 (2003)].

Après administration, l'énantiomère R présenterait une AUC (aire sous courbe) supérieure au racémique et une moindre variabilité des taux plasmatiques.

Le modafinil est utilisé dans le traitement de la somnolence diurne excessive associée à une narcolepsie avec ou sans catalepsie. La somnolence diurne excessive se caractérise par une difficulté à rester éveillé et une augmentation des endormissements survenant à des moments inopportuns. La dose initiale recommandée est de 200 mg par jour administrée en une seule prise le matin, ou en deux prises matin et midi selon la réponse du patient. Les doses peuvent être augmentées jusqu'à 600 mg pour les patients présentant une réponse insuffisante.

Le problème des formes commerciales disponibles actuellement réside dans la persistance de l'effet bien au-delà de la période souhaitée par le patient. Cette rémanence de la vigilance est finalement de nature à perturber le cycle normal de sommeil du patient et induit même une insomnie.

Le modafinil a également été utilisé avec succès chez l'enfant dans le traitement de l'ADHD (attention déficit hyperactivity disorder).

Dans toutes ces pathologies, on observe au niveau des signes cliniques une forte variabilité et il s'avère donc nécessaire de procéder à un ajustement thérapeutique individuel.

Un objet de la présente invention est de mettre à la disposition du patient une nouvelle forme médicamenteuse orale de S modafinil possédant une biodisponibilité augmentée comparativement au racémique et une durée d'action raccourcie.

L'un des objectifs est également de fournir une formulation capable de répondre à la forte variabilité inter individuelle et donc de mettre à la disposition du patient une formulation homothétique permettant un ajustement aisé de la dose administrée.

Un autre objet de l'invention est de mettre à la disposition du patient une préparation thérapeutique permettant un effet thérapeutique très rapide comparativement au racémique et comparativement à l'énantiomère S administré seul.

Différents documents de l'art antérieur décrivent l'utilisation du modafinil dans le traitement de la dépendance à la cocaïne ; ainsi, la demande de brevet US2001/0034373 décrit l'utilisation de dose journalière efficace de moins de 100 mg de modafinil, et plus particulièrement de 1 à 75 mg. Le texte n'apporte aucun enseignement sur les données de formulation ni données pharmacocinétiques.

La demande internationale PCT WO99/25329 mentionne l'utilisation de dérivés benzhydryl sulfonyle, dont le modafinil, pour réduire l'effet de somnolence induit par les traitements aux opiacés. Le document ne décrit pas non plus précisément les compositions pharmaceutiques ni d'effets pharmacocinétiques.

La demande de brevet US2004/06532 mentionne l'utilisation d'une forme pharmaceutique comportant entre 250 et 450 mg de modafinil par unité de prise thérapeutique. La présente invention concerne quant à elle des formes pharmaceutiques de S modafinil dosées à 100 mg de principe actif, voire moins.

La demande de brevet US2009/0123545 relate une association de composés de modafinil où l'énantiomère S est en concentration supérieure à 50%. Ces compositions sont utilisées pour augmenter l'état de vigilance, d'alerte, et plus globalement pour augmenter la stimulation du SNC (système nerveux central). Les données pharmaceutiques mentionnées enseignent que la composition en S modafinil est supérieure à 80% et que la demi vie est inférieure à 4 heures. La présente invention concerne pour sa part des préparations spécifiquement formulées de manière à obtenir une action rapide, inférieure ou égale à 1 heure, et une courte durée d'activité, demi vie inférieure à 2 heures.

Le texte WO2007/01362 décrit lui aussi des compositions à 100 mg de S modafinil mais nous apprend que l'effet induit est persistant jusque 4 heures.

L'intérêt de la présente invention est de posséder une action très rapide tout en étant fugace.

Ainsi la présente invention concerne des formes thérapeutiques orales de S Modafinil se présentant sous la forme de comprimés, sachets ou bien sous la forme de gélules dosés entre 25 et 200 mg de principe actif et préférentiellement de 50 à 100 mg.

Les formulations ici présentées sont homothétiques et donc sont identiques quels que soient les dosages administrés au patient, ce qui concourt à diminuer la forte variabilité observée.

Le modafinil se présente sous forme d'une poudre blanche cristalline pratiquement insoluble dans l'eau et partiellement soluble dans le méthanol et l'acétone. Il en résulte une faible biodisponibilité du modafinil ; on l'estime à environ 40%. En effet, la solubilité du principe actif étant trop faible, la biodisponibilité absolue n'a pas pu être déterminée.

Les compositions ici décrites ont été spécifiquement développées de manière à obtenir *in vitro* une dissolution très rapide et dans tous les cas supérieure à celle obtenue avec une forme commercialisée sur le marché; une méthode de dissolution discriminante a donc été spécifiquement développée et a permis de sélectionner les excipients et procédés de fabrication.

Un procédé d'une nouvelle formulation faisant l'objet de la présente invention, utilise la technologie du CO₂ supercritique, basée sur le pouvoir solvant du CO₂ qui est modulable à volonté selon les conditions de pression et de température qu'on lui applique. A l'état supercritique (plus de 74 bars et de 31°C), le CO₂ possède des propriétés très particulières. Le fluide obtenu est caractérisé par une grande diffusivité (de l'ordre de celle des gaz), ce qui lui confère une bonne aptitude à la diffusion, et une densité élevée qui le dote d'une capacité de transport et d'extraction importante.

Un fluide supercritique présente un autre avantage par rapport aux autres solvants : sa solubilité change selon que l'on fait varier sa température ou sa pression. On peut ainsi faire en sorte qu'il soit un solvant pour certaines substances à un moment donné, et plus du tout l'instant d'après. Cela facilite la récupération de la substance qui a été dissoute.

Il est apparu que le modafinil présente une solubilité acceptable dans le CO₂.

L'étape suivante a consisté à pulvériser le principe actif dissous sur un support inerte, puis à étudier la granulométrie des particules obtenues ainsi que la forme cristalline de ces mêmes particules.

Différents essais ont été réalisés, tout d'abord en utilisant du lactose anhydre comme support inerte.

Différents paramètres ont ensuite été modifiés :
- changement du support inerte (lactose anhydre remplacé par du mannitol),
- augmentation du taux de charge jusque 30% de charge de principe actif,
- utilisation du S modafinil,
- solvant supercritique autre que le CO₂.

Les essais sont récapitulés dans le tableau ci-dessous en utilisant du S modafinil et du mannitol :

| Essai | Quantité de modafinil extrait (g) | Solubilité (g/g) | Quantité de formulation collectée (g) | Rendement de collecte (%) | Taux de charge théorique (%) |
|---|---|---|---|---|---|
| Formulations oréparées avec CO₂ supercritique | | | | | |
| Formulation 1 | 10,0 | 1,0.E-04 | 29,54 | 96 | 32,65 |
| Formulation 2 | 9,5 | 9,3.E-05 | 29,40 | 98 | 31,52 |
| Formulation 3 | 9,7 | 9,9.E-05 | 29,62 | 98 | 32,18 |
| Formulations préparées avec un autre solvant | | | | | |
| Formulation 4 | 11,4 | 2,5.E-04 | 28,81 | 86 | 31,38 |
| Formulation 5 | 11,5 | 1,9.E-04 | 26,68 | 80 | 31,66 |
| Formulation 6 | 11,5 | 2,7.E-04 | 30,36 | 91 | 34,27 |

Les préparations obtenues précédemment ont été formulées de manière à obtenir des comprimés dosés à 2 mg de S modafinil, ces comprimés étant destinés à être administrés au rat lors d'une étude pharmacocinétique.

Le procédé utilisé est le suivant :
- après pesée de chacun des composants, les excipients sont introduits successivement par ordre pondéral croissant dans un mélangeur
- le mélange est ensuite tamisé pour éliminer les amas éventuels
- le mélange obtenu est ensuite compacté puis calibré sur une grille d'ouverture 1,25mm 250 tours/minute.
- la compression sur machine à comprimer SVIAC est ensuite effectuée.

### Formule réalisée

**DASC00512**

| **Matières Premières** | **g** | **%** |
|---|---|---|
| Echantillon 4 | 5,000 | 55,64 |
| Aérosil 200 | 1,250 | 13,91 |
| PVP XL | 0,358 | 3,99 |
| PVP K30 | 0,765 | 8,51 |
| Pearlitol 400 DC | 1,523 | 16,95 |
| Stéarate de magnésium | 0,090 | 1,00 |
| **TOTAL** | 8,987 | 100,00 |

Les résultats de dissolution apparaissent sur la figure suivante dans laquelle on compare le Modiodal, la formulation 4 (poudre), et l'échantillon DASC 00512 (comprimés avec dissolution en paniers).

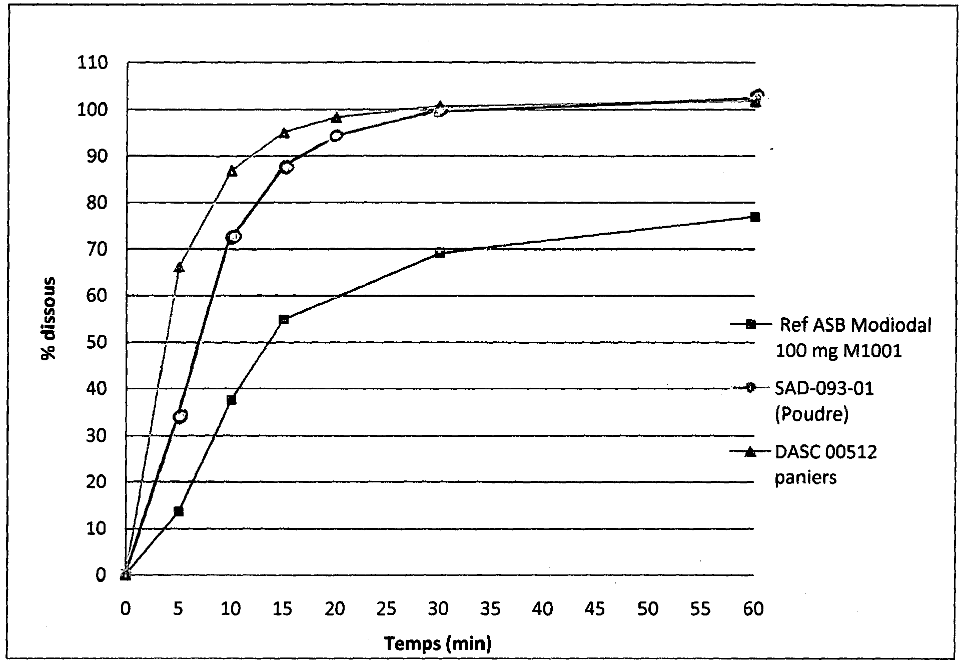

Un autre type de formulation, à savoir des gélules semi solides, a également été réalisé et testé.

**Formulation 1**

| ***Lot : DASC00176-2 Composants*** | ***Formule de fabrication (g)*** | ***Quantité par gélule (mg)*** | ***Bilan en %*** |
|---|---|---|---|
| ASC (2xbroyé) = énantiomère S | 20.00 | 4.00 | 28.6 |
| Lipoxol 400 MED | 25.00 | 5.00 | 35.7 |
| Polysorbate 80 | 25.00 | 5.00 | 35.7 |
| **TOTAL** | **/** | **14.00** | **100.0** |

**Formulation 2**

| ***Lot : DASC00177-2 Composants*** | ***Formule de fabrication (g)*** | ***Quantité par gélule (mg)*** | ***Bilan en %*** |
|---|---|---|---|
| ASC (2xbroyé) = énantiomère S | 20.00 | 4.00 | 28.6 |
| DUB GPE 810 | 25.00 | 5.00 | 35.7 |
| Polysorbate 80 | 25.00 | 5.00 | 35.7 |
| **TOTAL** | **/** | **14.00** | **100.0** |

**Formulation 3**

| ***Lot : DASC00174-2 Composants*** | ***Formule de fabrication (g)*** | ***Quantité par gélule (mg)*** | ***Bilan en* %** |
|---|---|---|---|
| ASC (2xbroyé) = énantiomère S | 20.00 | 4.00 | 28.6 |
| Lipoxol 400 MED | 16.67 | 3.33 | 23.8 |
| DUB GPE 810 | 16.67 | 3.33 | 23.8 |
| Polysorbate 80 | 16.67 | 3.33 | 23.8 |
| **TOTAL** | **/** | **13.99** | **100.0** |

**Formulation 4**

| ***Lot : DASB00175-2 Composants*** | ***Formule de fabrication (g)*** | ***Quantité par gélule (mg)*** | ***Bilan en %*** |
|---|---|---|---|
| ASB (2xbroyé) = racémique | 20.00 | 4.00 | 28.6 |
| Lipoxol 400 MED | 16.67 | 3.33 | 23.8 |
| DUB GPE 810 | 16.67 | 3.33 | 23.8 |
| Polysorbate 80 | 16.67 | 3.33 | 23.8 |
| **TOTAL** | **/** | **13.99** | **100.0** |

A titre d'exemple, le procédé de fabrication de la formulation 1 va maintenant être donné, étant entendu que les autres formulations suivent le même procédé, à savoir : pesée des matières premières et double broyage du principe actif (comme pour les comprimés), dispersion de la poudre obtenue dans les excipients et répartition en gélules.
1- Dans un bêcher de 100 ml, Incorporer 25 g de Lipoxol 400 MED (Polyéthylène glycol 400)
2- Mettre sous agitation à 250 tours/mn.
3- Incorporer à température ambiante 25 g de Polysorbate 80 et laisser agiter pendant 2 minutes
4- Incorporer lentement 20 g de principe actif broyé
5- Laisser agiter 10 minutes à 350 tours/mn après dispersion.

On obtient une dispersion blanchâtre homogène et légèrement visqueuse à température ambiante titrée à 285,7 mg/g de principe actif.

Cette dispersion sera incorporée dans la gélule semi-solide type HIBAR et maintenue sous agitation à 200 tours/mn à l'aide d'un agitateur muni d'une hélice marine afin d'effectuer le remplissage de gélules gélatine.

La température de la trémie et de l'injecteur sera réglée à 27° C. on procédera ensuite à la fermeture des gélules puis à leur mise en stabilité.

La figure suivante permet de comparer les profils de dissolution des références et des formulations.

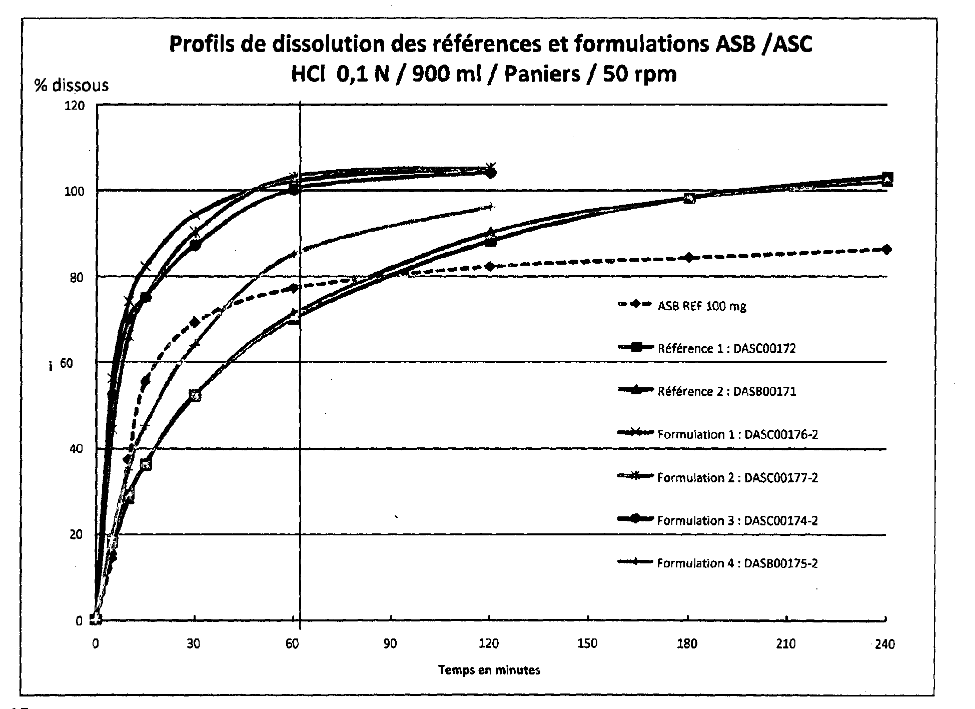

Il ressort de cette figure que les quatre formulations en gélules présentent un profil de dissolution plus rapide que la référence ASB dosée à 100 mg.

Toutes les formulations en gélules présentent une dissolution supérieure à celle des références internes.

Parmi les formulations, celle à base d'ASB présente une dissolution plus lente que les trois formulations ASC dont les profils de dissolution sont identiques.

On observe en comparant les profils de dissolution obtenus que l'utilisation de l'énantiomère S de l'ASB et de la formulation spécifique ici présentée permet d'obtenir des résultats de dissolution *in vitro* très rapides aux alentours de 90% en 30 minutes comparativement aux produits de référence et au mélange racémique dans une formulation identique.

Ainsi, qu'il s'agisse de la formulation de S modafinil obtenue par le procédé du fluide supercritique, ou bien des gélules semi solides, on arrive au même résultat concernant le profil pharmacocinétique, à savoir une libération extrêmement rapide, inférieure à une heure, et plus précisément de l'ordre de 30 minutes, et un effet fugace, c'est-à-dire, la disparition de l'effet persistant du modafinil en matière de vigilance accrue dans les 4 heures suivant l'absorption de la composition pharmaceutique par le patient.

Cette double caractéristique en termes de libération très rapide et d'effet fugace est particulièrement intéressante dans le traitement des cocaïnomanes.

Ainsi que cela a déjà été mentionné, la dépendance à la cocaïne est devenue un fléau majeur de santé publique au niveau mondial pour lequel il n'existe pas actuellement de traitements de fond efficaces. Les patients bénéficient en effet de désintoxications répétées et coûteuses en milieu hospitalier, à l'issue desquelles ils rechutent presque inéluctablement.

Le traitement se limite d'ailleurs à des psychotropes non spécifiques pendant les désintoxications et diverses psychothérapies d'efficacité non démontrée sont effectuées entre ces périodes de désintoxication.

De nombreux traitements ont été testés avec un succès limité et le modafinil est celui qui paraît le plus prometteur aux experts internationaux.

En effet, la cible élective de la cocaïne est le système dopaminergique des régions du cerveau impliquées dans les phénomènes d'addiction. La cocaïne inhibe les transporteurs membranaires neuronaux de la dopamine (DA) et amplifie ainsi les réponses dopaminergiques.

Le modafinil est un psychostimulant non amphétaminique dont le mécanisme d'action est incomplètement connu mais a une composante dopaminergique majeure, précisément au niveau des DAT. Ses autres effets, noradrénergique et glutamatergique, sont également reliés aux actions non dopaminergiques de la cocaïne.

Ces similitudes neuropharmacologiques ont amené à considérer le modafinil comme un traitement substitutif potentiel de la dépendance à la cocaïne.

Le modafinil a été testé dans les années 2000 dans plusieurs essais cliniques chez des patients cocaïnomanes. Ces essais, réalisés aux Etats-Unis, avaient un effectif de patients limité et ont donné des résultats encourageants mais dissociés, l'efficacité étant retrouvée sur certains mais pas tous les critères d'évaluation cliniques et biologiques.

L'interprétation de ces résultats positifs mais encore insuffisants a fait appel au concept de substitution imparfaite. Le modafinil mime les effets pharmacologiques de la cocaïne, mais son profil pharmacocinétique est trop différent de celui de la cocaïne pour réaliser une substitution suffisamment « dissuasive » de la sensation de manque (*craving*) et de la consommation de cocaïne chez le sujet dépendant.

L'objectif est d'obtenir un début d'action très rapide en 15 à 30 minutes permettant de réduire la fenêtre du *craving* et de permettre au sujet de résister à la compulsion addictive. Le produit obtenu réalise une substitution beaucoup plus performante que celle de la molécule native.

La forme pharmaceutique selon l'invention aboutit à un profil pharmacocinétique permettant de répondre à cette double exigence : un début d'action très rapide et un effet éveillant du S modafinil limité, inférieur à 4 heures et préférentiellement inférieur à 2 heures, afin d'éviter le risque d'insomnie liée à l'action éveillante prolongée du R modafinil. En effet, la couverture de la période vespérale, un des moments sensibles du *craving* et des rechutes, nécessite une prise du produit en fin d'après-midi. Il n'y aura donc pas d'inconvénient à ce que le patient prenne une dose de composition selon l'invention à ce moment de la journée. Ladite forme pharmaceutique constitue donc bien une forme thérapeutique de substitution pour les cocaïnomanes ; elle se présentera sous forme non aqueuse, c'est-à-dire sous forme solide ou semi solide, chaque dose unitaire contiendra de 25 à 200 mg de S modafinil, et préférentiellement de 50 à 100 mg.

Préférentiellement, la forme pharmaceutique selon l'invention se présentera sous forme orale, par exemple des comprimés ou des gélules, évitant ainsi les risques sanitaires liés aux prises par injections.

## Revendications

1. Composition pharmaceutique à base de modafinil S pour son utilisation dans le traitement de substitution des cocaïnomanes, **caractérisé en ce que** modafinil S est obtenu par la technologie du fluide supercritique, ledit modafinil S étant absorbé à la surface de granules réalisées en un support inerte.

2. Composition pour son utilisation selon la revendication 1 **caractérisée en ce que** ledit fluide est du CO₂.

3. Composition pour son utilisation selon l'une des revendications 1 ou 2 **caractérisée en ce que** ledit support est du lactose anhydre.

4. Composition pour son utilisation selon l'une des revendications 1 ou 2 **caractérisée en ce que** ledit support est du mannitol.

5. Composition pour son utilisation selon l'une quelconque des revendications 1 à 4 **caractérisée en ce que** ladite composition se présente sous forme de comprimés.

6. Composition pour son utilisation selon la revendication 5 **caractérisée en ce que** ledit comprimé contient de 25 à 200 mg de modafinil S.

7. Composition pour son utilisation selon la revendication 6 **caractérisée en ce que** ledit comprimé contient de 50 à 100 mg de modafinil S.

## Patentansprüche

1. Pharmazeutische Zusammensetzung auf der Basis von Modafinil S für ihre Verwendung bei der Substitutionsbehandlung von Kokainabhängigen, **dadurch gekennzeichnet, dass** Modafinil S anhand der Technologie des superkritischen Fluids erhalten wird, wobei das Modafinil S auf der Oberfläche von in einem inerten Träger realisierten Granulat absorbiert wird.

2. Zusammensetzung für ihre Verwendung nach Anspruch 1, **dadurch gekennzeichnet, dass** das Fluid CO₂ ist.

3. Zusammensetzung für ihre Verwendung nach einem der Ansprüche 1 oder 2, **dadurch gekennzeichnet, dass** der Träger wasserfreie Laktose ist.

4. Zusammensetzung für ihre Verwendung nach einem der Ansprüche 1 oder 2, **dadurch gekennzeichnet, dass** der Träger Mannitol ist.

5. Zusammensetzung für ihre Verwendung nach einem der Ansprüche 1 bis 4, **dadurch gekennzeichnet, dass** die Zusammensetzung in Form von Tabletten vorliegt.

6. Zusammensetzung für ihre Verwendung nach Anspruch 5, **dadurch gekennzeichnet, dass** die Tablette 25 bis 200 mg Modafinil S enthält.

7. Zusammensetzung für ihre Verwendung nach Anspruch 6, **dadurch gekennzeichnet, dass** die Tablette 50 bis 100 mg Modafinil S enthält.

## Claims

1. A S-modafinil-based pharmaceutical composition for use in the substitution treatment of cocaine addicts, **characterized in that** S-modafinil is obtained using supercritical fluid technology, said S-modafinil being absorbed on the surface of granules produced in an inert carrier.

2. The composition for use according to claim 1, **characterized in that** said fluid is CO₂.

3. The composition for use according to one of claims 1 or 2, **characterized in that** said carrier is anhydrous lactose.

4. The composition for use according to one of claims 1 or 2, **characterized in that** said carrier is mannitol.

5. The composition for use according to any one of claims 1 to 4, **characterized in that** said composition is in tablet form.

6. The composition for use according to claim 5, **characterized in that** said tablet contains from 25 to 200 mg of S-modafinil.

7. The composition for use according to claim 6, **characterized in that** said tablet contains from 50 to 100 mg of S-modafinil.
